# EUROPEAN PATENT SPECIFICATION

(11) **EP 1 725 536 B1**
(45) Date of publication and mention of the grant of the patent: **15.10.2008**
(21) Application number: 05716710.8
(22) Date of filing: 16.02.2005
(51) Int. Cl.: C07D 233/28

(54) **IMIDAZOLINE DERIVATIVES HAVING CB1-ANTAGONISTIC ACTIVITY**
IMIDAZOLINDERIVATE MIT CB1-ANTAGONISTISCHER WIRKUNG
DERIVES DE L'IMIDAZOLINE PRESENTANT UNE ACTIVITE ANTAGONISTE CB1

(30) Priority: 19.02.2004 EP 04100656; 19.02.2004 US 545484 P
(43) Date of publication of application: 29.11.2006
(73) Proprietor: Solvay Pharmaceuticals B.V., 1381 CP Weesp (NL)
(72) Inventor: LANGE, Josephus H. M., p/o IPSI DEPARTMENT, NL-1381 CP Weesp (NL); KRUSE, Cornelis G., p/o IPSI DEPARTMENT, NL-1381 CP Weesp (NL); VAN STUIVENBERG, Herman H., p/o IPSI DEPARTMENT, NL-1381 CP Weesp (NL)
(74) Representative: Verhage, Marinus
(86) International application number: PCT/EP2005/050680
(87) International publication number: WO 2005/080345

(56) References cited:
- WO-A-03/026647
- WO-A-03/027076
- WO-A-03/078413
- WO-A-03/101969
- LANGE J H M ET AL: "SYNTHESIS, BIOLOGICAL PROPERTIES, AND MOLECULAR MODELING INVESTIGATIONS OF NOVEL 3,4-DIARYLPYRAZOLINES AS POTENT AND SELECTIVE CB1 CANNABINOID RECEPTOR ANTAGONISTS" JOURNAL OF MEDICINAL CHEMISTRY, AMERICAN CHEMICAL SOCIETY, US, vol. 47, no. 3, 30 December 2003 (2003-12-30), pages 627-643, XP001188902 ISSN: 0022-2623
- KHANNA I K ET AL: "SELECTIVE CYCLOOXYGENASE-2 INHIBITORS: HETEROARYL MODIFIED 1,2-DIARYLIMIDAZOLES ARE POTENT, ORALLY ACTIVE ANTIINFLAMMATORY AGENTS" JOURNAL OF MEDICINAL CHEMISTRY, AMERICAN CHEMICAL SOCIETY. WASHINGTON, US, vol. 43, no. 16, 2000, pages 3168-3185, XP001026112 ISSN: 0022-2623

## Description

The present invention relates to 1,2,4-tri-substituted imidazoline derivatives as CB₁ antagonists, to methods for the preparation of these compounds and to novel intermediates useful for the synthesis of said imidazoline derivatives. The invention also relates to the use of a compound disclosed herein for the manufacture of a medicament giving a beneficial effect. A beneficial effect is disclosed herein or apparent to a person skilled in the art from the specification and general knowledge in the art. The invention also relates to the use of a compound of the invention for the manufacture of a medicament for treating or preventing a disease or condition. In embodiments of the invention specific compounds disclosed herein are used for the manufacture of a medicament useful in the treatment of disorders in which cannabinoid receptors are involved, or that can be treated via manipulation of those receptors.

In WO 03/0226647, 4,5-dihydro-1H-pyrazole derivatives having CB₁-antagonistic activity were disclosed. Synthesis, biological properties and molecular modeling investigations of those compounds was also published by Lange et al. (J.Med. Chem., 2004, 47, 627-643). 1H-imidazoles and thiazole derivatives having CB₁ agonistic, CB₁ partial agonistic or CB₁ antagonistic activity were disclosed in WO 03/027076 and WO 03/078413 respectively.

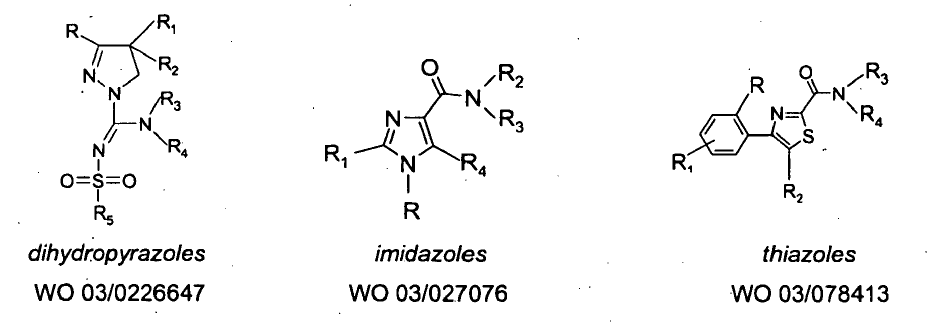

Multisubstituted imidazoline derivatives are known from WO 03/101954 and WO 03/101969. The compounds described therein, are potent inhibitors of transcription factor NF-KB, making them useful in the treatment of certain types of tumors. Said imidazoline derivatives also have potent activities as anti-inflammatory agents and antibiotics, leading to an additional array of indications in which they are likely to be of therapeutic interest, including inflammatory and infectious diseases. The compounds described in the abovementioned patent applications were not demonstrated to have any affinity for cannabinoid receptors, and therefore unlikely to be of therapeutic value in disorders in which these cannabinoid receptors are involved.

The goal of the present invention was to identify imidazoline derivatives with potent activity as cannabindid-CB₁ receptor modulators, whilst maintaining essentially the physico-chemical properties that make some imidazoline derivatives useful therapeutic agents.

It has now surprisingly been found that potent antagonism or inverse agonism of cannabinoid-CB₁ receptors is present in the novel 4,5-dihydro-1H-imidazole derivatives of the formula (I): 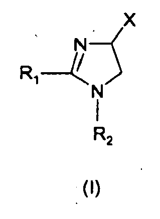 wherein:
- R₁ and R₂ independently represent phenyl, thienyl or pyridyl which groups may be substituted with 1, 2 or 3 substituents Y, which can be the same or different, from the group branched or linear C₁₋₃-alkyl or C₁₋₃-alkoxy, phenyl, hydroxy, chloro, bromo, fluoro, iodo, trifluoromethyl, trifluoromethylthio, trifluoromethoxy, carboxyl, trifluoromethylsulfonyl, cyano, carbamoyl, sulfamoyl and acetyl, or R₁ and/or R₂ represent naphtyl,
- X represents one of the subgroups (i) or (ii), 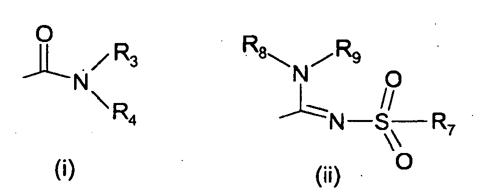 wherein:
   - R₃ represents a hydrogen atom or a branched or linear C₁₋₃ alkyl group,
   - R₄ represents a branched or linear C₁₋₈ alkyl or C₃₋₈-cycloalkyl-C₁₋₂-alkyl group, branched or linear C₁₋₈ alkoxy, C₃₋₈ cycloalkyl, C₅₋₁₀ bicycloalkyl, C₆₋₁₀ tricycloalkyl, which groups may contain one or more heteroatoms from the group (O, N, S) and which groups may be substituted with a hydroxy group, 1-3 methyl groups, an ethyl group or 1-3 fluoro atoms, or R₄ represents a phenyl, phenoxy, benzyl, phenethyl or phenylpropyl group, optionally substituted on their phenyl ring with 1-3 substituents Y, wherein Y has the abovementioned meaning, or R₄ represents a pyridyl or thienyl group, or R₄ represents a group NR₅R₆ wherein R₅ and R₆ - together with the nitrogen atom to which they are attached -form a saturated or unsaturated, monocyclic or bicyclic, heterocyclic group having 4 to 10 ring atoms, which heterocyclic group contains one or two heteroatoms from the group (O N, S) and which heterocyclic group may be substituted with a branched or linear C₁₋₃ alkyl, phenyl, hydroxy or trifluoromethyl group or a fluoro atom, or
      R₃ and R₄ - together with the nitrogen atom to which they are attached - form a saturated or unsaturated, monocyclic or bicyclic, heterocyclic group having 4 to 10 ring atoms, which heterocyclic group contains one or two heteroatoms from the group (O, N, S) and which heterocyclic group may be substituted with a branched or linear C₁₋₃ alkyl, phenyl, amino, hydroxy or trifluoromethyl group or a fluoro atom,
- R₇ represents a benzyl, phenyl, thienyl or pyridyl group, which groups may be substituted on their aromatic ring with 1, 2, 3 or 4 substituents Y, wherein Y has the meaning as indicated above, which can be the same or different, or R₇ represents C₁₋₈ branched or linear alkyl, C₃₋₈ alkenyl, C₃₋₁₀ cycloalkyl, C₅₋₁₀ bicycloalkyl, C₆₋₁₀ tricycloalkyl or C₅₋₈ cycloalkenyl or R₇ represents naphtyl or R₇ represents an amino group or R₇ represents a C₁₋₈ dialkylamino group, a C₁₋₈ monoalkylamino group or a saturated or unsaturated, monocyclic or bicyclic, heterocyclic group having 4 to 10 ring atoms, which heterocyclic group contains 1 or 2 nitrogen atoms and which heterocyclic group may contain 1 heteroatom from the group (O, S) and which heterocyclic group may be substituted with a branched or linear C₁₋₃ alkyl, phenyl, hydroxy or trifluoromethyl group or a fluoro atom,
- R₈ represents a hydrogen atom or a methyl group,
- R₉ represents a hydrogen atom or a methyl, ethyl or methoxy group
and tautomers, stereoisomers and salts thereof.

At least one centre of chirality is present (at the C₄ position of the imidazoline moiety) in the compounds of the formula (I). The invention relates to racemates, mixtures of diastereomers as well as the individual stereoisomers of the compounds having formula (I). The invention also relates to the E isomer, Z isomer and E/Z mixtures of compounds having formula (I).

The invention particularly relates to compounds having formula (I) 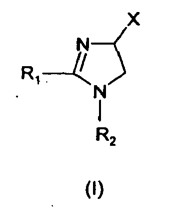 wherein:
- R₁ and R₂ independently represent phenyl, which phenyl group may be substituted with 1, 2 or 3 substituents Y, which can be the same or different, from the group branched or linear C₁₋₃-alkyl or C₁₋₃-alkoxy, phenyl, hydroxy, chloro, bromo, fluoro, iodo, trifluoromethyl, trifluoromethylthio, trifluoromethoxy, carboxyl, trifluoromethylsulfonyl, cyano, carbamoyl, sulfamoyl and acetyl, or R₁ and/or R₂ represent naphtyl, thienyl or pyridyl,
- X.represents one of the subgroups (i) or (ii), 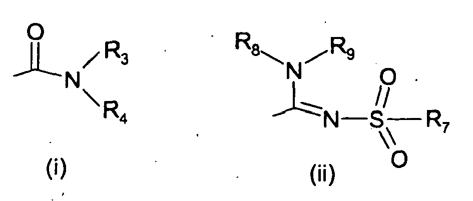 wherein:
   - R₃ represents a hydrogen atom,
   - R₄ represents a branched or linear C₁₋₈ alkyl, branched or linear C₁₋₈ alkoxy or C₃₋₈ cycloalkyl group, which groups may be substituted with a hydroxy group, 1-3 methyl groups, an ethyl group or 1-3 fluoro atoms, or R₄ represents a phenyl, phenoxy, pyridyl or thienyl group, or R₄ represents a group NR₅R₆ wherein R₅ and R₆ - together with the nitrogen atom to which they are attached -form a saturated or unsaturated, monocyclic or bicyclic, heterocyclic group having 4 to 10 ring atoms, which heterocyclic group contains one or two heteroatoms from the group (O N, S) or
      R₃ and R₄ - together with the nitrogen atom to which they are attached - form a saturated or unsaturated, monocyclic or bicyclic, heterocyclic group having 4 to 10 ring atoms, which heterocyclic group contains one or two heteroatoms from the group (O, N, S) and which heterocyclic group may be substituted with a methyl, hydroxy or trifluoromethyl group or a fluoro atom,
- R₇ represents a phenyl group, which phenyl group may be substituted on its aromatic ring with 1, 2, 3 or 4 substituents Y, wherein Y has the meaning as indicated above, which can be the same or different, or R₇ represents C₁₋₈ branched or linear alkyl, C₃₋₁₀ cycloalkyl or C₅₋₁₀ bicycloalkyl, or R₇ represents naphtyl or R₇ represents a amino group or R₇ represents a C₁₋₈ dialkylamino group, a C₁₋₈ monoalkylamino group or a saturated or unsaturated, monocyclic or bicyclic, heterocyclic group having 4 to 10 ring atoms, which heterocyclic group contains 1 or 2 nitrogen atoms and which heterocyclic group may contain 1 heteroatom from the group (O, S) and which heterocyclic group may be substituted with a branched or linear C₁₋₃ alkyl or hydroxy group,
- R₈ represents a hydrogen atom,
- R₉ represents a hydrogen atom and tautomers, stereoisomers and salts thereof.

Due to the potent CB₁ antagonistic activity the compounds according to the invention are suitable for use in the treatment of psychiatric disorders such as psychosis, anxiety, depression, attention deficits, memory disorders, cognitive disorders, appetite disorders, obesity, in particular juvenile obesity and drug induced obesity, addiction, impulse control disorders, appetence, drug dependence and neurological disorders such as neurodegenerative disorders, dementia, dystonia, muscle spasticity, tremor, epilepsy, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuropathic pain disorders, and other diseases involving cannabinoid neurotransmission, including the treatment of septic shock, glaucoma, cancer, diabetes, emesis, nausea, asthma, respiratory diseases, gastrointestinal disorders, gastric ulcers, diarrhoea, cardiovascular disorders, atherosclerosis, liver cirrhosis and sexual disorders.

The cannabinoid receptor modulating activity of the compounds of the invention makes them particularly useful in the treatment of obesity, juvenile obesity and drug induced obesity, when used in combination with lipase inhibitors. Specific examples of compounds which can be used in such combination preparations are (but not restricted to) the synthetic lipase inhibitor orlistat, lipase inhibitors isolated from micro organisms such as lipstatin (from *Streptomyces toxytricini*)*,* ebelactone B (from *Streptomyces aburaviensis*), synthetic derivatives of these compounds, as well as extracts of plants known to possess lipase inhibitory activity, for instance extracts of *Alpinia officinarum* or compounds isolated from such extracts like 3-methylethergalangin (from *A. officinarum*)*.*

### GENERAL ASPECTS OF SYNTHESES

The synthesis of compounds having formula (I) wherein X represents subgroup (i) is outlined in Scheme 1. intermediates having general formula (II) can be obtained according to methods known, see for example: I. K. Khanna et al., J. Med. Chem. 2000, 43, 3168-3185; I. K. Khanna et al., J. Med. Chem. 1997, 40, 1634-1647; WO 03/027076 or WO 03/040107. Intermediates having general formula (IV) can be obtained according to methods known, see for example: I. K. Khanna et al., J. Med. Chem. 2000, 43, 3168-3185.
Carboxamidine derivatives of general formula (II) can be reacted with 2-chloroacrylonitrile (III) to give a 4,5-dihydro-1H-imidazole derivative of general formula (IV). This reaction is preferably carried out in the presence of a base such as N,N-diisopropylethylamine. The obtained derivatives of general formula (IV) can be esterified with an alcohol R₁₀-OH to give a 4,5-dihydro-1H-imidazole derivative of general formula (V), wherein R₁₀ represents a branched or linear C₁₋₆ alkyl group or a benzyl group. This reaction is preferably carried out under acidic conditions. A compound of general formula (V) can react with an amine R₃R₄NH, preferably In the presence of trimethylaluminum (Me₃Al) to give a compound of formula (I), wherein X represents subgroup (i) and R₃ and R₄ have the meaning as given above on page 2. Additional information on trimethylaluminum Al(CH₃)₃ promoted amidation reactions of esters can be found in: J. I. Levin, E. Turos, S. M. Weinreb, Synth Commun. (1982), 12, 989-993.

Alternatively, a compound of general formula (V) can be hydrolysed to the corresponding carboxylic acid derivative of general formula (VI), wherein R₁₁ represents H or an earth alkali metal, in particular Li, Na or K. Alternatively, the compound of general formula (VI) can be reacted with a chlorinating agent such as thionylchloride to give the corresponding acid chloride. The compound of general formula (VI) can be reacted with an amine R₃R₄NH to give a compound of formula (I), wherein X represents subgroup (i) and R₃ and R₄ have the meaning as given above on page 2, *via* activating and coupling methods such as formation of an active ester, or in the presence of a so-called coupling reagent, such as for example, DCC, HBTU, BOP (benzotriazol-1-yloxytris(dimethylamino)phosphonium hexafluorophosphate) and the like. Additional information on activating and coupling methods of amines to carboxylic acids can be found in:
a) M. Bodanszky and A. Bodanszky: The Practice of Peptide Synthesis, Springer-Verlag, New York, 1994; ISBN: 0-387-57505-7;
b) K. Akaji et al., Tetrahedron Lett. (1994), 35, 3315-3318);
c) F. Albericio et al., Tetrahedron Lett. (1997), 38, 4853-4856).

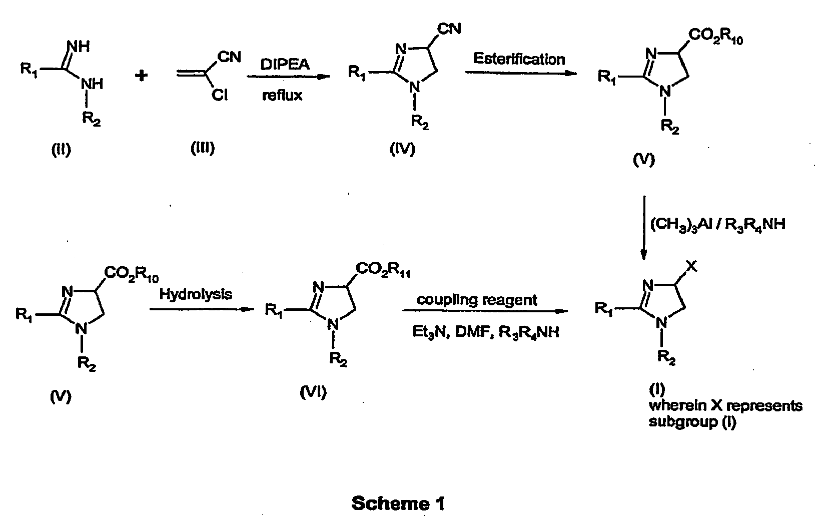

The synthesis of compounds having formula (I) wherein X represents subgroup (ii) is outlined in Scheme 2.

An intermediate of general formula R₇SO₂NH₂ is either commercially available or can be prepared via standard synthetic methodology, for example from the corresponding compound R₇SO₂Cl (see for example; McManus et al., J. Med. Chem. 1965, 8, 766). A compound of general formula (IV) can be reacted with a compound of general formula R₇SO₂NH₂ in the presence of a Lewis acid such as for example AlMe₃ in an inert organic solvent such as benzene to give a compound of general formula (I) wherein X represents subgroup (ii) and R₁, R₂ and R₇ have the meaning as given above on the pages 1-3 and wherein R₈ and R₉ represent a hydrogen atom. A compound of general formula (V) may be reacted with a compound of general formula R₇SO₂NH₂ to give a compound of general formula (VII). This reaction is preferably carried out in the presence of a strong non -nucleophilic base. A compound of general formula (VII) may be reacted with a chlorinating reagent in a chloroimidation reaction and subsequently treated with an amine R₈R₉NH to give a compound of formula (I), wherein X represents subgroup (ii).

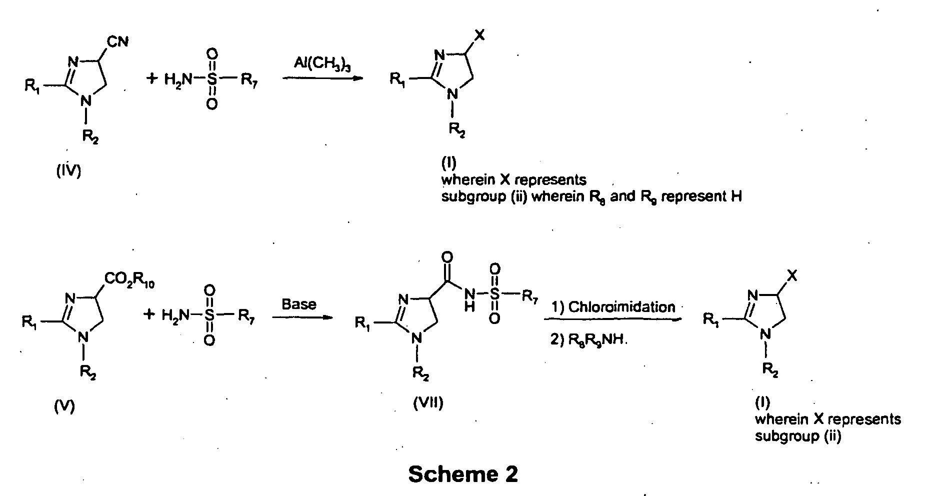

The selection of the particular synthetic method depends on factors such as the compatibility of functional groups with the reagents used, the possibility to use protecting groups, catalysts, activating and coupling reagents and the ultimate structural features present in the final compound being prepared.

According to these procedures the following compounds can be prepared. They are intended to further illustrate the invention in more detail.

### PHARMACEUTICAL PREPARATIONS

The compounds of the invention can be brought into forms suitable for administration by means of usual processes using auxiliary substances such as liquid or solid carrier material. The pharmaceutical compositions of the invention may be administered enterally, orally, parenterally (intramuscularly or Intravenously), rectally or locally (topically). They can be administered in the form of solutions, powders, tablets, capsules (including microcapsules), ointments (creams or gel) or suppositories. Suitable excipients for such formulations are the pharmaceutically customary liquid or solid fillers and extenders, solvents, emulsifiers, lubricants, flavorings, colorings and/or buffer substances. Frequently used auxiliary substances which may be mentioned are magnesium carbonate, titanium dioxide, lactose, mannitol and other sugars, talc, lactoprotein, gelatin, starch, cellulose and its derivatives, animal and vegetable oils such as fish liver oil, sunflower, groundnut or sesame oil, polyethylene glycol and solvents such as, for example, sterile water and mono- or polyhydric alcohols such as glycerol.

Compounds of the present invention are generally administered as pharmaceutical compositions which are important and novel embodiments of the invention because of the presence of the compounds, more particularly specific compounds disclosed herein. Types of pharmaceutical compositions that may be used include but are not limited to tablets, chewable tablets, capsules, solutions, parenteral solutions, suppositories, suspensions, and other types disclosed herein or apparent to a person skilled in the art from the specification and general knowledge In the art. In embodiments of the invention, a pharmaceutical pack or kit is provided comprising one or more containers filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

### PHARMACOLOGICAL METHODS

### In vitro affinity for cannabinoid-CB₁ receptors

The affinity of the compounds of the invention for cannabinoid CB, receptors can be determined using membrane preparations of Chinese hamster ovary (C HO) cells in which the human cannabinoid CB₁ receptor is stably transfected in conjunction with [³H]CP-55,940 as radioligand. After incubation of a freshly prepared cell membrane preparation with the [³H]-ligand, with or without addition of compounds of the invention, separation of bound and free ligand is performed by filtration over glassfiber filters. Radioactivity on the filter is measured by liquid scintillation counting.

### In vitro cannabinoid-CB₁ receptor antagonism

In vitro CB₁ receptor antagonism can be assessed with the human CB₁ receptor cloned in Chinese hamster ovary (CHO) cells. CHO cells are grown in a Dulbecco's Modified Eagle's medium (DMEM) culture medium, supplemented with 10% heat-inactivated fetal calf serum. Medium is aspirated and replaced by DMEM, without fetal calf serum, but containing [^{a}H]-arachidonic add and incubated overnight in a cell culture stove (5% CO₂/95% air, 37°C; water-saturated atmosphere). During this period [³H]-arachidonic add is incorporated in membrane phosphol ipids. On the test day, medium is aspirated and cells are washed three times using 0.5 mL DMEM, containing 0.2% bovine serum albumin (BSA). Stimulation of the CB₁ receptor by WIN 55,212-2 leads to activation of PLA₂ followed by release of [³H]-arachidonic acid into the medium. This WIN 55,212-2-induced release is concentration-dependently antagonized by CB₁ receptor antagonists.

### In vivo cannabinoid-CB₁ receptor antagonism

In vivo CB₁ antagonism can be assessed with the CP-55,940-induced hypotension test in rat. Male normotensive rats (225-300 g; Harlan, Horst The Nethertands) are anaesthetized with pentobarbital (80 mg/kg *i.p*.). Blood pressure is measured, via a cannula inserted into the left carotid artery, by means of a Spectramed DTX-plus pressure transducer (Spectramed B.V., Bilthoven, The Netherlands). After amplification by a Nihon Kohden Carrier Amplifier (Type AP-621G; Nihon Kohden B.V., Amsterdam, The Netherlands), the blood pressure signal is registered on a personal computer (Compaq Deskpro 386s), by means of a Po-Ne-Mah data-acquisition program (Po-Ne-Mah Inc, Storrs, USA). Heart rate is derived from the pulsatile pressure signal. All compounds are administered orally as a microsuspension in 1% methylcellulose 30 minutes before induction of the anesthesia which is 60 minutes prior to administration of the CB₁ receptor agonist CP-55,940. The injection volume is 10 ml/kg. After haemodynamic stabilization the CB₁ receptor agonist CP-55,940 (0.1 mg/kg *i.v.*) is administered and the hypotensive effect established. (Wagner, J. A.; Jarai, Z.; Batkai. S.; Kunos, G. Hemodynamic effects of cannabinoids: coronary and cerebral vasodilation mediated by cannabinoid CB1 receptors. Eur.J.Pharmacol. 2001, 423, 203-10).

Pharmaceutically acceptable salts may be obtained using standard procedures well known in the art, for example by mixing a compound of the present invention with a suitable acid, for instance an inorganic acid such as hydrochloric acid, or with an organic acid.

### DOSE

The affinity of the compounds of the invention for cannabinoid receptors was determined as described above. From the binding affinity measured for a given compound of formula (1), one can estimate a theoretical lowest effective dose. At a concentration of the compound equal to twice th e measured K₁-value, 100% of the cannabinoid receptors likely will be occupied by the compound. Converting that concentration to mg of compound per kg of patient yields a theoretical lowest effective dose, assuming ideal bioavailability. Pharmacokinetic, pharmacodynamic, and other considerations may alter the dose actually administered to a higher or lower value. The dosage expediently administered is 0.001 ― 1000 mg/kg, preferably 0.1-100 mg/kg of patient's bodyweight.

### EXAMPLE 1: SYNTHESES OF SPECIFIC COMPOUNDS

### Compounds 1-2

Part A: A magnetically stirred mixture of N-(4-chlorophenyl)-2,4-dichlorobenzenecarboxamidine (10.0 gram, 0.033 mol), 2-chloroacrylonitrile (5.7 gram, 0.065 mol) and N,N-diisopropylethylamine (DIPEA) (12.5 ml, 0.069 mol) in tetrahydrofuran (150 ml) is heated at reflux temperature for 40 hours (N₂ atmosphere). After cooling to room temperature the mixture is concentrated in vacuo. The residue is dissolved in a mixture of dichloromethane and water (200 ml/200 ml). The dichloromethane layer is collected, dried over MgSO₄, filtered and concentrated in vacuo. The residue is recrystallised from ethanol/water to give 1-(4-chlorophenyl)-2-(2,4-dichlorophenyl)-4,5-dihydro-1H-imidazole-4-carbonitrile (11.23 gram, 97 % yield).¹H-NMR (400 MHz, CDCl₃): δ4.28 (dd, J = 10 and 8 Hz, 1 H), 4.36 (t, J = 10 Hz, 1H), 5.07 (dd, J = 10 and 8 Hz, 1 H), 6.68 (br d, J = 8 Hz, 2H), 7.16 (br d, J = 8 Hz, 2H), 7.32-7.36 (m, 2H), 7.45 (d, J = 8 Hz, 1H).

Part B: Acetyl chloride (17.76 ml, 0.25 mol) is slowly added to ethanol (1 l) to give solution A. 1-(4-chlorophenyl)-2-(2,4-dichlorophenyl)-4,5-dihydro-1H-imidazole-4-carbonitrile (17.52 gram, 0.05 mol) is added in one portion to solution A. After cooling to room temperature the mixture is stirred for another 40 hours and concentrated in vacuo. The residue is dissolved in dichloromethane and washed (3x) with aqueous (5 %) NaHCO₃. The dichloromethane layer is separated, dried over MgSO₄, filtered and concentrated in vacuo to give ethyl 1-(4-chlorophenyl)-2-(2,4-dichlorophenyl)-4,5-dihydro-1H-imidazole-4-carboxylate (18.0 gram, 90 % yield) as a brown oil that slowly solidifies on standing. ¹H-NMR (400 MHz, CDCl₃): δ 1.34 (t, J = 7 Hz, 3H), 4.15 (dd, J = 10 and 8 Hz, 1H), 4.22-4.41 (m, 3H), 4.91 (dd, J = 10 and 8 Hz, 1H), 6.66 (br d, J = 8 Hz, 2H), 7.11 (br d, J = 8 Hz, 2H), 7.30 (dd, J = 8 and 2 Hz, 1H), 7.33 (d, J = 2 Hz, 1H), 7.46 (dd, J = 8 Hz, 1H).

Part C: To a magnetically stirred solution of exo-2-aminobicyclo[2.2.1]heptane (0.67 ml, 0.009 mol) in anhydrous dichloromethane (10 ml) is added trimethylaluminum (5.4 ml of a 2N solution in hexane, 0.0108 mol) and the resulting solution is stirred for 20 minutes at room temperature. A solution of ethyl 1-(4-chlorophenyl)-2-(2,4-dichlorophenyl)4,5-dihydro-1H-imidazole-4-carboxylate (2.385 g, 0.006 mol) in anhydrous dichloromethane (10 ml) is slowly added and the resulting mixture is reacted at 40°C for 40 hours (N₂ atmosphere). After cooling to room temperature the mixture is quenched with aqueous (5 %) NaHCO₃ and extracted with dichloromethane. The dichloromethane layer is separated, dried over MgSO₄, filtered and concentrated in vacuo to give a crude yellow syrup (2.58 gram) which is further purified with flash chromatography (silica gel, ethyl acetate/petroleum ether = 8/2 (v/v)) to give the faster moving 1-(4-chlorophenyl)-2-(2.4-dichlorophenyl)-N-(exo-2-bicyclo[2.2.1]heptyl)-4,5-dihydro-1H-imidazole-4-carboxamide (dia-stereomer A) (0.70 gram, 25 % yield) and the slower moving 1-(4-chlorophenyl)-2-(2,4-dichlorophenyl)-N-(exo-2-bicyclo[2.2.1]heptyl)-4,5-dihydro -1H-imidazole-4-carboxamide (diastereomer B) (0.69 gram, 25 % yield).

Diastereomer A: ¹H-NMR (400 MHz, CDCl₃):δ 1.10-1.58 (m, 7H), 1.76-1.84 (m, 1 H), 2.26-2.30 (m, 2H), 3.74-3.82 (m, 1H), 4.27 (d, J ∼ 10 Hz, 2H), 4.78 (t, J - 10 Hz, 1 H), 6.65 (br d, J = 8 Hz, 2H), 6.70-6.78 (m, 1H), 7.12 (br d, J = 8 Hz, 2H), 7.29 (br s, 2H), 7.40 (br s, 1 H).

Diastereomer B:¹H-NMR (400 MHz, CDCl₃): δ 1.10-1.56 (m, 7H), 1.78-1.85 (m, 1H), 2.17-220 (m, 1H), 2.26-2.30 (m, 1H), 3.76-3.82 (m, 1H), 4.25-4.30 (m, 2H), 4.78 (dd, J = 10 and 8 Hz, 1H), 6.66 (br d, J = 8Hz, 2H), 6.80 (brd, J - 7Hz, 1H), 7.11 (br d, J = 8 Hz, 2H), 7.30 (br s, 2H), 7.41 (br s, 1H).

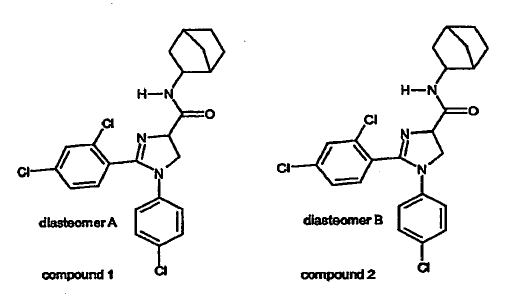

### Compounds 3 and 4

Part A: A mixture of ethyl 1-(4-chlorophenyl)-2-(2,4-dichlorophenyl)-4,5-dihydro-1H-imidazole-4-carboxylate (3.97 g, 0.01 mol) in methanol/water is reacted with LiOH (1.3 gram, 0.054 mol) at room temperature for 16 hours. The resulting mixture is concentrated in vacuo to give crude lithium 1-(4-chloraphenyl)-2-(2,4-dichlorophenyl)-4,5-dihydro-1H-imidazole-4-carboxylate (4.7 gram).

Part B: A mixture of the crude lithium 1-(4-chlorophenyl)-2-(2,4-dichlorophenyl)-4,5-dihydro-1H-imidazole-4-carboxylate (1.0 gram, - 0.0027 mol), benzotriazol-1-yloxytris(dimethylamino)phosphonium hexafluorophos-phate (BOP) (1.2 gram, 0.0027 mol), 1-aminopiperidine (0.3 gram, 0.003 mol) and triethylamine (1 ml) in DMF (30 ml) is stirred at room temperature for 16 hours. After concentration in vacuo, water is added and the resulting mixture is extracted (2x) with dichloromethane. The dichloromethane layers are collected, dried over MgSO₄, filtered and concentrated in vacuo to give a residue that is further purified by flash chromatography (silicagel, dichloromethane/methanol = 95/5 (v/v)) to give 1-(4-chlorophenyl)-2-(2,4-dichlorophenyl)-N-(piperidin-1-yl)-4,5-dihydro-1H-imidazole-4-carboxamide (380 mg, 31 % yield). Melting point 113-116 °C. ¹H-NMR (200 MHz, CDC1₃):δ 1.33-1.48 (m, 2H),1.60-1.80 (m, 4H), 2.68-2.82 (m, 4H), 4.28-4.35 (m, 2H), 4.84 (dd, J = 11 and 9 Hz, 1H), 6.65 (br d, J = 8 Hz, 2H), 7.11 (br d, J = 8 Hz, 2H), 7.23-7.33 (m, 2H), 7.41 (d, J = 2 Hz, 1 H), 7.57 (br s, 1H).

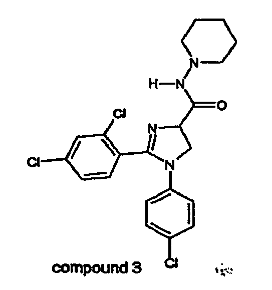

In an analogous manner compound 4 was prepared:

**Compound 4:** 1-(4-Chlorophenyl)-2-(2,4-dichlorophenyl)-N-cyclohexyl-4,5-dihydro-1H-imidazote-4-carboxamide. Melting point 127-129 °C. ¹H-NMR (200 MHz, CDCl₃): δ1.04-2.03 (m, 10H), 3.73-3.92 (m, 1H), 4.23-4.33 (m, 2H), 4.81 (t, J ∼ 10 Hz, 1 H), 6.66 (br d, J = 8 Hz, 2H), 6.79 (br d, J - 7 Hz, 1H), 7.12 (br d, J = 8 Hz, 2H), 7.25-7.32 (m, 2H), 7.41 (br s, 1H).

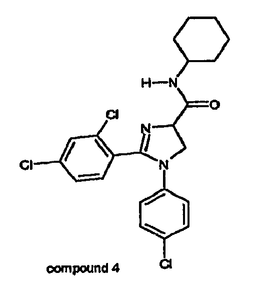

### Compounds 5 - 8

Part A: To a suspension of 4-chlorobenzenesulfonamide (0.45 gram, 0.00236 mol) in benzene (5 ml) is dropwise added trimethylaluminum (1.2 ml of a 2N solution in toluene, 0.0024 mol) to give a clear solution which is stirred at room temperature for 1 hour. 1-(4-Chlorophenyl)-2-(2,4-dichlorophenyl)-4,5-dihydro-1H-imidazole-4-carbonitrile (0.55 gram, 0.00157 mol) is added and the resulting mixture is heated at 90 °C for 16 hours. After cooling to room temperature a mixture of methanol/water (8/2 (v/v)) is slowly added, the solids are removed by filtration and washed with chloroform (50 ml). The filtrate is concentrated in vacuo. The residue is triturated with n-pentane and twice recrystallised from methanol to give 1-(4-chlorophenyl)-2-(2,4-dichlorophenyl)-N-[(4-chlorophenyl)sulfonyl]-4,5-dihydro-1H-imidazole-4-carboxamidine (0.435 gram, 51 % yield). Melting point 165-166 °C. ¹H-NMR (200 MHz, CDCl₃): δ 4.11-4.35 (m, 2H), 4.94 (dd, J =12 and 10 Hz, 1H), 6.63 (br d, J = 8 Hz, 2H), 7.12 (br d, J = 8 Hz, 2H), 7.22-7.52 (m, 6H), 7.90 (br d, J = 8 Hz, 2H), 8.10-8.20 (m,1H).

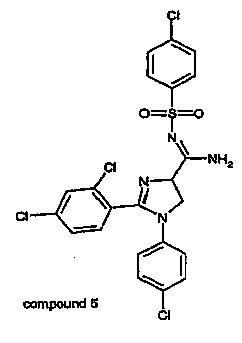

In an analogous manner the compounds having formula (I) listed below have been prepared:

**Compound 6:** 1-(4-Chlarophenyl)-2-(2,4-dichlorophenyl)-N-[(4-fluorophenyl)-sulfonyl]-4,5-dihydro-1H-imidazole-4-carboxamidine. Melting point 172-175 °C. ¹H-NMR (200 MHz, CDCl₃): δ 4.12-4.35 (m, 2H), 4.93 (dd, J =12 and 10 Hz, 1H), 6.63 (br d, J = 8 Hz, 2H), 7.08-7.43 (m, 8H), 7.90-8.02 (m, 2H), 8.10-8.20 (m, 1H).

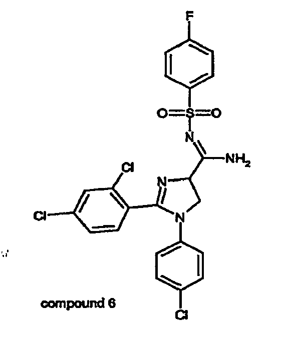

**Compound 7:** 2-(4-Chlorophenyl)-N-(dimethylaminosulfonyl)-1-phenyl-4,5-dihydro-1H-imidazole-4-carboxamidine. Melting point 136-139 °C. ¹H-NMR (200 MHz, CDCl₃): δ 2.79 (s, 6H), 4.20-4.40 (m, 2H), 4.97 (t, J - 10 Hz, 1H), 6.83 (br d, J = 8 Hz, 2H), 7.05-7.50 (m, 8H), 7.80-7.90 (m, 1H).

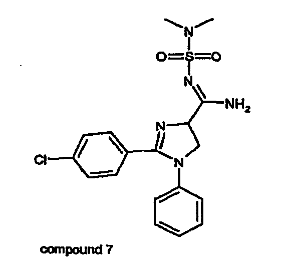

**Compound 8:** 1-(4-chlorophenyl)-2-(2,4-dichlorophenyl)-N-(dimethylaminosul-fonyl)-4,5-dihydro-1H-imidazole-4-carboxamidine. Melting point: 146-147 °C.

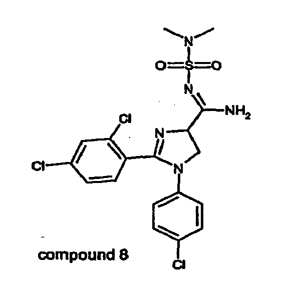

### EXAMPLE 2: FORMULATIONS AS USED IN ANIMAL STUDIES

### Formulation of compound 1:

For oral (*p.o*.) administration: to the desired quantity (0.5-15 mg) of the compound given above as 'Compound 1' in a glass tube, some glass beads were added and the substance was milled by vortexing for 2 minutes. After addition of 1 ml of a solution of 1% methylcellulose in water, the compound was suspended by vortexing for 10. minutes. For concentrations up and above 1 mg/ml remaining particles in the suspension were further suspended by using an ultrasonic bath.

### EXAMPLE 3: PHARMACOLOGICAL TESTRESULTS

Cannabinoid receptor affinity and functional *in vitro* data data obtained according to the protocols given above are shown in the table below.

**Table 1: pharmacological data**

| | Human cannabinoid-CB₁ receptor | |
|---|---|---|
| | *in vitro* affinity | *In vitro* antagonism |
| Compound nr | pk₁ value | pA₂-value |
| | | (arachidonic acid release) |
| | | |
| Compound 1 | 7.7 | - |
| Compound 2 | 7.0 | - |
| Compound 4 | 7.0 | 7.7 |
| Compound 8 | 6.8 | - |

## Claims

1. Compounds of the general formula (I) 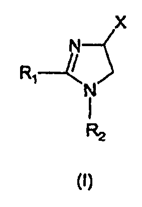 wherein:
- R₁ and R₂ independently represent phenyl, thienyl or pyridyl which groups may be substituted with 1; 2 or 3 substituents Y, which can be the same or different, from the group branched or linear C₁₋₃-alkyl or C₁₋₃-alkoxy, phenyl, hydroxy, chloro, bromo, fluoro, iodo, trifluoromethyl, trifluoromethylthio, trifluoromethoxy, carboxyl, trifluoromethylsulfonyl, cyano, carbamoyl, sulfamoyl and acetyl, or R₁ and/or R₂ represent naphtyl,
- X represents one of the subgroups (i) or (ii), 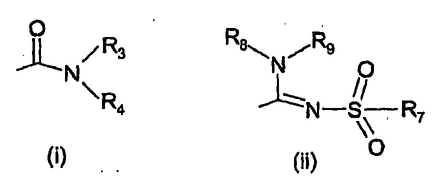 wherein:
- R₃ represents a hydrogen atom or a branched or linear C₁₋₃ alkyl group,
- R₄ represents a branched or linear C₁₋₈ alkyl or C₁₋₈-cycloalkyl-C₁₋₂-alkyl group, branched or linear C₁₋₈ alkoxy, C₃₋₈ cycloalkyl, C₅₋₁₀ bicycloalkyl, C₆₋₁₀ tricycloalkyl, which groups may contain one or more heteroatoms from the group (O, N, S) and which groups may be substituted with a hydroxy group, 1-3 methyl groups, an ethyl group or 1-3 fluoro atoms, or R₄ represents a phenoxy, benzyl, phenethyl or phenylpropyl group, optionally substituted on their phenyl ring with 1-3 substituents Y, wherein Y has the abovementioned meaning, or R₄ represents a pyridyl or thienyl group, or R₄ represents a group NR₅R₆ wherein
R₅ and R₆ - together with the nitrogen atom to which they are attached form a saturated or unsaturated, monocyclic or bicyclic, heterocyclic group having 4 to 10 ring atoms, which heterocyclic group contains one or two heteroatoms from the group (O, N, S) and which heterocyclic group may be substituted with a branched or linear C₁₋₃ alkyl, phenyl, hydroxy or trifluoromethyl group or a fluoro atom, or
R₃ and R₄ - together with the nitrogen atom to which they are attached - form a saturated or unsaturated, monocyclic or bicyclic, heterocyclic group having 4 to 10 ring atoms, which heterocyclic group contains one or two heteroatoms from the group (O, N, S) and which heterocyclic group may be substituted with a branched or linear C₁₋₃ alkyl, phenyl, amino, hydroxy or trifluoromethyl group or a fluoro atom,
- R₇ represents a benzyl, phenyl, thienyl or pyridyl group, which groups may be substituted on their aromatic ring with 1, 2, 3 or 4 substituents Y, wherein Y has the meaning as indicated above, which can be the same or different, or R₇ represents C₁₋₈ branched or linear alkyl, C₃₋₈ alkenyl, C₃₋₁₀ cycloalkyl, C₅₋₁₀ bicycloalkyl, C₆₋₁₀ tricycloalkyl or C₅₋₈ cycloalkenyl or R₇ represents naphtyl or R₇ represents a amino group or R₇ represents a C₁₋₈ dialkylamino group, a C₁₋₈ monoalkylamino group or a saturated or unsaturated, monocyclic or bicyclic, heterocyclic group having 4 to 10 ring atoms, which heterocyclic group contains 1 or 2 nitrogen atoms and which heterocyclic group may contain 1 heteroatom from the group (O, S) and which heterocyclic group may be substituted with a branched or linear C₁₋₃ alkyl, phenyl, hydroxy or trifluoromethyl group or a fluoro atom,
- R₈ represent a hydrogen atom or a methyl group,
- R₉ represents a hydrogen atom or a methyl, ethyl or methoxy group,
and tautomers, stereoisomers and salts thereof

2. Compounds as claimed in claim 1 of the general formula (I) 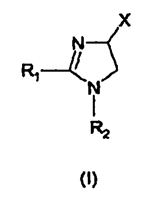 wherein:
- R₁ and R₂ independently represent phenyl, which phenyl group may be substituted with 1, 2 or 3 substituents Y, having the meanings as given in claim 1, or R₁ and/or R₂ represent naphtyl, thienyl or pyridyl,
- X represents one of the subgroups (i) or (ii), 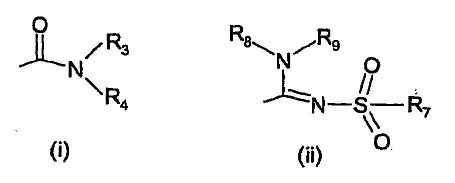 wherein:
- R₃ represents a hydrogen atom,
- R₄ represents a branched or linear C₁₋₈ alkyl, branched or linear C₁₋₈ alkoxy or C₃₋₄ cycloalkyl group, which groups may be substituted with a hydroxy group, 1-3 methyl groups, an ethyl group or 1-3 fluoro atoms, or R₄ represents a phenoxy, pyridyl or thienyl group, or R₄ represents a group NR₅R₆ wherein
R₅ and R₆ - together with the nitrogen atom to which they are attached form a saturated or unsaturated, monocyclic or bicyclic, heterocyclic group having 4 to 10 ring atoms, which heterocyclic group contains one or two heteroatoms from the group (O, N, S) or
R₃ and R₄ - together with the nitrogen atom to which they are attached - form a saturated or unsaturated, monocyclic or bicyclic, heterocyclic group having 4 to 10 ring atoms, which heterocyclic group contains one or two heteroatoms from the group (O, N, S) and which heterocyclic group may be substituted with a methyl, hydroxy or trifluoromethyl group or a fluoro atom,
- R₇ represents a phenyl group, which phenyl group may be substituted on its aromatic ring with 1, 2, 3 or 4 substituents Y, wherein Y has the meaning as indicated above, which can be the same or different, or R₇ represents C₁₋₈ branched or linear alkyl, C₃₋₁₀ cycloalkyl or C₅₋₁₀ bicycloalkyl, or R₇ represents naphtyl or R₇ represents a amino group or R₇ represents a C₁₋₈ dialkylamino group, a C₁₋₈ monoalkylamino group or a saturated or unsaturated, monocyclic or bicyclic, heterocyclic group having 4 to 10 ring atoms, which heterocyclic group contains 1 or 2 nitrogen atoms and which heterocyclic group may contain 1 heteroatom from the group (O, S) and which heterocyclic group may be substituted with a branched or linear C₁₋₃ alkyl or hydroxy group,
- R₈ represent a hydrogen atom,
- R₉ represents a hydrogen atom
and tautomers, stereoisomers and salts thereof.

3. The compound according to claim 1 which is:
1-(4-chlorophenyl)-2-(2,4-dichlorophenyl)-N-(exo-2-bicyclo[2.2.1]heptyl)-4,5-dihydro-1H-imidazole-4-carboxamide (diastereomer A)
1-(4-chlorophenyl)-2-(2,4-dichlorophenyl)-N-(exo-2-bicyclo[2.2.1]heptyl)4,5-dihydro-1 H-imidazole-4-carboxamide (diastereomer B)
1-(4-chlorophenyl)-2-(2,4-dichlorophenyl)-N-(piperidin-1-yl)-4,5-dihydro-1 H-imidazole-4-carboxamide
1-(4-Chlorophenyl)-2-(2,4-dichlorophenyl)-N-cyclohexyl-4,5-dihydro-1H-imidazole-4-carboxamide
1-(4-chlorophenyl)-2-(2,4-dichlorophenyl)-N-[(4-chlorophenyl)sulfanyl]-4,5-dihydro-1H-imidazole-4-carboxamidine
1-(4-Chlorophenyl)-2-(2,4-dichlorophenyl)-N-[(4-fluorophenyl)-sulfonyl]-4,5-dihydro-1 H-imidazole-4-carboxamidine
2-(4-Chlorophenyl)-N-(dimethylaminosulfonyl)-1-phenyl-4,5-dihydro-1H-imidazole-4-carboxamidine
1-(4-chlorophenyl)-2-(2,4-dichlorophenyl)-N-(dimethylaminosul-fonyl)-4,5-dihydro-1 H-imidazole-4-carboxamidine

4. Pharmaceutical compositions comprising, in addition to a pharmaceutically acceptable carrier and/or at least one pharmaceutically acceptable auxiliary substance, a pharmacologically active amount of at least one compound as claimed in any of the claims 1-3, or a salt thereof, as an active ingredient.

5. A compound as claimed in any of the claims 1-3, or a salt thereof, for use in medicine

6. Use of a compound as claimed in any of the claims 1-3, for the preparation of a pharmaceutical composition for the treatment of psychosis, anxiety, depression, attention deficits, memory disorders, cognitive disorders, appetite disorders, obesity, in particular juvenile obesity and drug induced obesity, addiction, impulse control disorders, appetence, drug dependence and neurological disorders such as neurodegenerative disorders, dementia, dystonia, muscle spasticity, tremor, epilepsy, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuropathic pain disorders, and other diseases involving cannabinoid neurotransmission, including the treatment of septic shock, glaucoma, cancer, diabetes, emesis, nausea, asthma, respiratory diseases, gastrointestinal disorders, gastric ulcers, diarrhoea, cardiovascular disorders, atherosclerosis, liver cirrhosis and sexual disorders.

7. Use of a compound of formula (I): 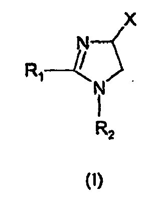 wherein:
- R₁ and R₂ have the meanings as given in claim 1, but may also independently represent methylsulfonyl,
- X represents the subgroup (i), 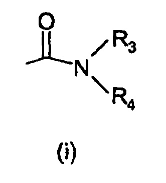 wherein:
- R₃ and R₄ have the meanings as given in claim 1, but in which R₄ may also represent a phenyl group, optionally substituted with 1-3 substituents Y, wherein Y has the meaning as given in claim 1,
for the preparation of a pharmaceutical composition for the treatment of psychosis, anxiety, depression, attention deficits, memory disorders, cognitive disorders, appetite disorders, obesity, in particular juvenile obesity and drug induced obesity, addiction, impulse control disorders, appetence, drug dependence and neurological disorders such as neurodegenerative disorders, dementia, dystonia, muscle spasticity, tremor, epilepsy, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, stroke, spinal cord injury, neuroinflammatory disorders, plaque sclerosis, viral encephalitis, demyelinisation related disorders, as well as for the treatment of pain disorders, including neuropathic pain disorders, and other diseases involving cannabinoid neurotransmission, including the treatment of septic shock, glaucoma, cancer, diabetes, emesis, nausea, asthma, respiratory diseases, gastrointestinal disorders, gastric ulcers, diarrhea, cardiovascular disorders, atherosclerosis, liver cirrhosis and sexual disorders.

8. Use as claimed in claim 6 **characterized in that** said disorders are eating disorders, in particular obesity, juvenile obesity and drug induced obesity.

9. Use of a compound as claimed in any of the claims 1-3 for the preparation of a pharmaceutical composition for the treatment of eating disorders, in particular obesity, juvenile obesity and drug induced obesity, **characterized in that** said pharmaceutical composition also contains at least one lipase inhibitor.

10. Use as claimed in claim 9, **characterized in that** said lipase inhibitor is orlistat or lipstatin.

## Patentansprüche

1. Verbindungen der allgemeinen Formel (I) 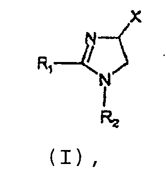 worin:
- R₁ und R₂ unabhängig Phenyl, Thienyl oder Pyridyl darstellen, welche Gruppen substituiert sein können mit 1, 2 oder 3 Substituenten Y, welche gleich oder unterschiedlich sein können, aus der Gruppe verzweigtes oder lineares C₁₋₃-Alkyl oder C₁₋₃-Alkoxy, Phenyl, Hydroxy, Chlor, Brom, Fluor, Iod, Trifluormethyl, Trifluormethylthio, Trifluormethoxy, Carboxyl, Trifluormethylsulfonyl, Cyano, Carbamoyl, Sulfamoyl und Acetyl, oder R₁ und/oder R₂ Naphthyl darstellen,
- X eine der Untergruppen (i) oder (ii) 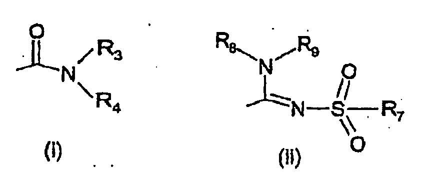
darstellt, worin
- R₃ ein Wasserstoffatom oder eine verzweigte oder lineare C₁₋₃-Alkylgruppe darstellt,
- R₄ eine verzweigte oder lineare C₁₋₈-Alkyl- oder C₃₋₈-Cycloalkyl-C₁₋₂-alkylgruppe, verzweigtes oder lineares C₁₋₈-Alkoxy, C₃₋₈-Cycloalkyl, C₅₋₁₀-Bicycloalkyl, C₆-₁₀-Tricycloalkyl darstellt, welche Gruppen ein oder mehrere Heteroatome aus der Gruppe (O, N, S) enthalten können und welche Gruppen mit einer Hydroxygruppe, 1-3 Methylgruppen, einer Ethylgruppe oder 1-3 Fluoratomen substituiert sein können, oder R₄ eine Phenoxy-, Benzyl-, Phenethyl- oder Phenylpropylgruppe darstellt, gegebenenfalls substituiert an ihrem Phenylring mit 1-3 Substituenten Y; worin Y die oben erwähnte Bedeutung hat, oder R₄ eine Pyridyl- oder Thienylgruppe darstellt, oder R₄ eine Gruppe NR₅R₆ darstellt, worin R5 und R₆ -zusammen mit dem Stickstoffatom, an welches sie gebunden sind - eine gesättigte oder ungesättigte, monocyclische oder bicyclische, heterocyclische Gruppe mit 4 bis 10 Ringatomen bilden, welche heterocyclische Gruppe ein oder zwei Heteroatome aus der Gruppe (O, N, S) enthält, und welche heterocyclische Gruppe substituiert sein kann mit einer verzweigten oder linearen C₁₋₃-Alkyl-, Phenyl-, Hydroxy- oder Trifluormethylgruppe oder einem Fluoratom, oder
R₃ und R₄ - zusammen mit dem Stickstoffatom, an welches sie gebunden sind - eine gesättigte oder ungesättigte, monocyclische oder bicyclische, heterocyclische Gruppe mit 4 bis 10 Ringatomen bilden, welche heterocyclische Gruppe ein oder zwei Heteroatome aus der Gruppe (O, N, S) enthält, und welche heterocyclische Gruppe substituiert sein kann mit einer verzweigten oder linearen C₁₋₃-Alkyl-, Phenyl-, Amino-, Hydroxy- oder Trifluormethylgruppe oder einem Fluoratom;
- R₇ eine Benzyl-, Phenyl-, Thienyl- oder Pyridylgruppe darstellt, welche Gruppen an ihrem aromatischen Ring substituiert sein können mit 1, 2, 3 oder 4 Substituenten Y, worin Y die Bedeutung wie oben angezeigt hat, welche gleich oder unterschiedlich sein können, oder R₇ für C₁₋₈-verzweigtes oder lineares Alkyl, C₃₋₈-Alkenyl, C₃₋₁₀-Cycloalkyl, C₅₋₁₀-Bicycloalkyl, C₆₋₁₀-Tricycloalkyl oder C₅₋₈-Cycloalkenyl steht oder R₇ Naphthyl darstellt oder R₇ eine Aminogruppe darstellt oder R₇ eine C₁₋₈-Dialkylaminogruppe, eine C₁₋₈-Monoalkylaminogruppe oder eine gesättigte oder ungesättigte, monocyclische oder bicyclische, heterocyclische Gruppe mit 4 bis 10 Ringatomen darstellt, welche heterocyclische Gruppe 1 oder 2 Stickstoffatome enthält und welche heterocyclische Gruppe 1 Heteroatom aus der Gruppe (O, S) enthalten kann und welche heterocyclische Gruppe substituiert sein kann mit einer verzweigten oder linearen C₁₋₃-Alkyl-, Phenyl-, Hydroxy- oder Trifluormethylgruppe oder einem Fluoratom,
- R₈ ein Wasserstoffatom oder eine Methylgruppe darstellt,
- R₉ ein Wasserstoffatom oder eine Methyl-, Ethyl- oder Methoxygruppe darstellt; und Tautomere, Stereoisomere und Salze davon.

2. Verbindungen wie in Anspruch 1 beansprucht der allgemeinen Formel (I) 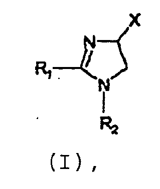 worin:
- R₁ und R₂ unabhängig Phenyl darstellen, welche Phenylgruppe substituiert sein kann mit 1, 2 oder 3 Substituenten Y mit den wie in Anspruch 1 gegebenen Bedeutungen, oder R₁ und/oder R₂ Naphthyl, Thienyl oder Pyridyl darstellen,
- X eine der Untergruppen (i) oder (ii) 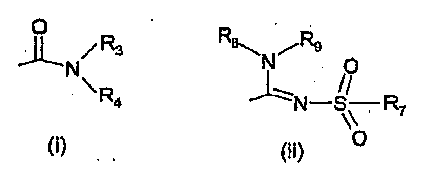
darstellt, worin
- R₃ ein Wasserstoffatom darstellt,
- R₄ eine verzweigte oder lineare C₁₋₈-Alkyl-, verzweigte oder lineare C₁₋₈-Alkoxy- oder C₃₋₈-Cycloalkylgruppe darstellt, welche Gruppen mit einer Hydroxygruppe, 1-3 Methylgruppen, einer Ethylgruppe oder 1-3 Fluoratomen substituiert sein können, oder R₄ eine Phenoxy-, Pyridyl- oder Thienylgruppe darstellt, oder R₄ eine Gruppe NR₅R₆ darstellt, worin R₅ und R₆ - zusammen mit dem Stickstoffatom, an welches sie gebunden sind - eine gesättigte oder ungesättigte, monocyclische oder bicyclische, heterocyclische Gruppe mit 4 bis 10 Ringatomen bilden, welche heterocyclische Gruppe ein oder zwei Heteroatome aus der Gruppe (O, N, S) enthält, oder
R₃ und R₄ - zusammen mit dem Stickstoffatom, an welches sie gebunden sind - eine gesättigte oder ungesättigte, monocyclische oder bicyclische, heterocyclische Gruppe mit 4 bis 10 Ringatomen bilden, welche heterocyclische Gruppe ein oder zwei Heteroatome aus der Gruppe (O, N, S) enthält, und welche heterocyclische Gruppe substituiert sein kann mit einer Methyl-, Hydroxy- oder Trifluormethylgruppe oder einem Fluoratom;
- R₇ eine Phenylgruppe darstellt, welche Phenylgruppe an ihrem aromatischen Ring substituiert sein kann mit 1, 2, 3 oder 4 Substituenten Y, worin Y die Bedeutung wie oben angezeigt hat, welche gleich oder unterschiedlich sein können, oder R₇ für C₁₋₈-verzweigtes oder lineares Alkyl, C₃₋₁₀-Cycloalkyl oder C₅₋₁₀-Bicycloalkyl steht, oder R₇ Naphthyl darstellt oder R₇ eine Aminogruppe darstellt oder R₇ eine C₁₋₈-Dialkylaminogruppe, eine C₁₋₈-Monoalkylaminogruppe oder eine gesättigte oder ungesättigte, monocyclische oder bicyclische, heterocyclische Gruppe mit 4 bis 10 Ringatomen darstellt, welche heterocyclische Gruppe 1 oder 2 Stickstoffatome enthält und welche heterocyclische Gruppe 1 Heteroatom aus der Gruppe (O, S) enthalten kann und welche heterocyclische Gruppe substituiert sein kann mit einer verzweigten oder linearen C₁₋₃-Alkyl- oder Hydroxygruppe,
- R₈ ein Wasserstoffatom darstellt,
- R₉ ein Wasserstoffatom darstellt;
und Tautomere, Stereoisomere und Salze davon.

3. Verbindung gemäß Anspruch 1, welche ist:
1-(4-Chlorphenyl)-2-(2,4-dichlorphenyl)-N-(exo-2-bicyclo[2.2.1]heptyl)-4,5-dihydro-1H-imidazol-4-carboxamid (Diastereomer A)
1-(4-Chlorphenyl)-2-(2,4-dichlorphenyl)-N-(exo-2-bicyclo[2.2.1]heptyl)-4,5-dihydro-1H-imidazol-4-carboxamid (Diastereomer B)
1-(4-Chlorphenyl)-2-(2,4-dichlorphenyl)-N-(piperidin-i-yl)-4,5-dihydro-1H-imidazol-4-carboxamid
1-(4-Chlorphenyl)-2-(2,4-dichlorphenyl)-N-cyclohexyl-4,5-dihydro-1H-imidazol-4-carboxamid
1-(4-Chlorphenyl)-2-(2,4-dichlorphenyl)-N-[(4-chlorphenyl)sulfonyl]-4,5-dihydro-1H-imidazol-4-carboxamidin
1-(4-Chlorphenyl)-2-(2,4-dichlorphenyl)-N-[(4-fluorphenyl)-sulfonyl]-4,5-dihydro-1H-imidazol-4-carboxamidin
2-(4-Chlorphenyl)-N-(dimethylaminosulfonyl)-1-phenyl-4,5-dihydro-1H-imidazol-4-carboxamidin
1-(4-Chlorphenyl)-2-(2,4-dichlorphenyl)-N-(dimethylaminosulfonyl)-4,5-dihydro-1H-imidazol-4-carboxamidin.

4. Pharmazeutische Zusammensetzungen, welche zusätzlich zu einem pharmazeutisch annehmbaren Träger und/oder mindestens einer pharmazeutisch annehmbaren Hilfssubstanz eine pharmakologisch wirksame Menge von mindestens einer Verbindung wie in einem der Ansprüche 1-3 beansprucht oder ein Salz davon als einen wirksamen Inhaltsstoff umfasst.

5. Verbindung wie in einem der Ansprüche 1-3 beansprucht, oder ein Salz davon, zur Verwendung in Medizin.

6. Verwendung einer Verbindung wie in einem der Ansprüche 1-3 beansprucht für die Herstellung einer pharmazeutischen Zusammensetzung für die Behandlung von Psychose, Angstzustand, Depression, Aufmerksamkeitsdefiziten, Gedächtnisstörungen, kognitiven Störungen, Appetitstörungen, Fettsucht, insbesondere juveniler Fettsucht und Arznei-, bzw. Drogen-induzierter Fettsucht, Sucht, Impulskontrollstörungen, Appetenz, Arznei- bzw. Drogenabhängigkeit, und neurologischen Störungen wie neurodegenerativen Störungen, Demenz, Dystonie, Muskelspastizität, Tremor, Epilepsie, Multiple Sklerose, traumatischer Hirnverletzung, Schlaganfall, Parkinson-Krankheit, Alzheimer-Krankheit, Epilepsie, Huntington-Krankheit, Tourette-Syndrom, zerebraler Ischämie, zerebraler Apoplexie, kraniozerebralem Trauma, Schlaganfall-, Rückenmarksverletzung, neuroentzündlichen Störungen, Plaque-Sklerose, viraler Enzephalitis, demyelinisierungsbezogenen Störungen, als auch für die Behandlung von Schmerzstörungen, einschließlich neuropathischen Schmerzstörungen, und weiteren Krankheiten, welche Cannabinoid-Neurotransmission einbeziehen, einschließlich der Behandlung von septischem Schock, Glaukom, Krebs, Diabetes, Erbrechen, Übelkeit, Asthma, Atemwegserkrankungen, gastrointestinalen Störungen, Magengeschwüren, Diarrhöe, kardiovaskulären Störungen, Atherosklerose, Leberzirrhose und sexuellen Störungen.

7. Verwendung einer Verbindung der Formel (I): 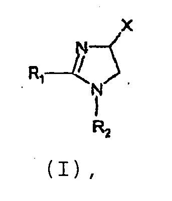 worin:
- R₁ und R₂ die wie in Anspruch 1 gegebenen Bedeutungen haben, aber auch unabhängig Methylsulfonyl darstellen können,
- X die Untergruppe (i) 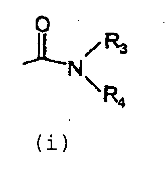
darstellt, worin:
- R₃ und R₄ die wie in Anspruch 1 gegebenen Bedeutungen haben, aber worin R₄ auch eine Phenylgruppe darstellen kann, gegebenenfalls substituiert mit 1-3 Substituenten Y, worin Y die wie in Anspruch 1 gegebene Bedeutung hat,
für die Herstellung einer pharmazeutischen Zusammensetzung für die Behandlung von Psychose, Angstzustand, Depression, Aufmerksamkeitsdefiziten, Gedächtnisstörungen, kognitiven Störungen, Appetitstörungen, Fettsucht, insbesondere juveniler Fettsucht und Arznei-, bzw. Drogen-induzierter Fettsucht, Sucht, Impulskontrollstörungen, Appetenz, Arznei- bzw. Drogenabhängigkeit, und neurologischen Störungen wie neurodegenerativen Störungen, Demenz, Dystonie, Muskelspastizität, Tremor, Epilepsie, Multiple Sklerose, traumatischer Hirnverletzung, Schlaganfall, Parkinson-Krankheit, Alzheimer-Krankheit, Epilepsie, Huntington-Krankheit, Tourette-Syndrom, zerebraler Ischämie, zerebraler Apoplexie, kraniozerebralem Trauma, Schlaganfall, Rückenmarksverletzung, neuroentzündlichen Störungen, Plaque-Sklerose, viraler Enzephalitis, demyelinisierungsbezogenen Störungen, als auch für die Behandlung von Schmerzstörungen, einschließlich neuropathischen Schmerzstörungen, und weiteren Krankheiten, welche Cannabinoid-Neurotransmission einbeziehen, einschließlich der Behandlung von septischem Schock, Glaukom, Krebs, Diabetes, Erbrechen, Übelkeit, Asthma, Atemwegserkrankungen; gastrointestinalen Störungen, Magengeschwüren, Diarrhöe, kardiovaskulären Störungen, Atherosklerose, Leberzirrhose und sexuellen Störungen.

8. Verwendung wie in Anspruch 6 beansprucht, **dadurch gekennzeichnet, dass** besagte Störungen Essstörungen, insbesondere Fettsucht, juvenile Fettsucht und Arznei- bzw. Drogen-induzierte Fettsucht sind.

9. Verwendung einer Verbindung wie in einem der Ansprüche 1-3 beansprucht für die Herstellung einer pharmazeutischen Zusammensetzung für die Behandlung von Essstörungen, insbesondere Fettsucht, juveniler Fettsucht und Arznei- bzw. Drogen-induzierter Fettsucht, **dadurch gekennzeichnet, dass** besagte pharmazeutische Zusammensetzung ebenfalls mindestens einen Lipasehemmer enthält.

10. Verwendung wie in Anspruch 9 beansprucht, **dadurch gekennzeichnet, dass** besagter Lipasehemmer Orlistat oder Lipstatin ist.

## Revendications

1. Composés répondant à la formule générale (I) 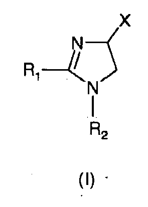 dans laquelle :
- R₁ et R₂ représentent indépendamment un groupe phényle, thiényle ou pyridyle, lesquels groupes peuvent être substitués par 1, 2 ou 3 substituants Y, qui peuvent être identiques ou différents, du groupe alkyle en C_{1 à 3} ramifié ou linéaire ou alcoxy en C_{1 à 3}, phényle, hydroxy, chloro, bromo, fluoro, iodo, trifluorométhyle, trifluorométhylthio, trifluorométhoxy, carboxyle, trifluorométhylsulfonyle, cyano, carbamoyle, sulfamoyle et acétyle, ou R₁ et/ou R₂ représente un groupe naphtyle,
- X représente l'un des sous-groupes (i) ou (ii), 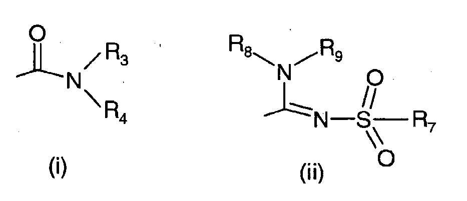
dans lesquels :
- R₃ représente un atome d'hydrogène ou un groupe alkyle en C_{1 à 3} ramifié ou linéaire,
- R₄ représente un groupe alkyle en C₁ à ramifié ou linéaire ou alkyle en C_{1 à 2} cycloalkyle en C_{3 à 8}, alcoxy en C_{1 à 8} ramifié ou linéaire, cycloalkyle en C_{3 à 8}, bicycloalkyle en C_{5 à 10,} tricycloalkyle en C_{6 à 10}, lesquels groupes peuvent contenir un ou plusieurs hétéroatomes parmi le groupe (O, N, S) et lesquels groupes peuvent être substitués par un groupe hydroxy, 1 à 3 groupes méthyle, un groupe éthyle, ou 1 à 3 atomes de fluor, ou R₄ représente un groupe phénoxy, benzyle, phénéthyle ou phénylpropyle, facultativement substitué sur leur cycle phényle par 1 à 3 substituants Y, où Y a la signification mentionnée ci-dessus, ou R₄ représente un groupe pyridyle ou thiényle, ou R₄ représente un groupe NR₅R₆ où
R₅ et R₆ - conjointement avec l'atome d'azote auquel ils sont attachés - forment un groupe hétérocyclique, monocyclique ou bicyclique saturé ou insaturé comportant 4 à 10 atomes de cycle, lequel groupe hétérocyclique contient un ou deux hétéroatomes parmi le groupe (O, N, S) et lequel groupe hétérocyclique peut être substitué par un groupe alkyle en C_{1 à 3} ramifié ou linéaire, phényle, hydroxy ou trifluorométhyle ou un atome de fluor, ou
R₃ et R₄ - conjointement avec l'atome d'azote auquel ils sont attachés - forment un groupe hétérocyclique, monocyclique ou bicyclique saturé ou insaturé comportant 4 à 10 atomes de cycle, lequel groupe hétérocyclique contient un ou deux hétéroatomes parmi le groupe (O, N, S) et lequel groupe hétérocyclique peut être substitué par un groupe alkyle en C_{1 à 3} ramifié ou linéaire, phényle, amino, hydroxy ou trifluorométhyle ou un atome de fluor, R₇ représente un groupe benzyle, phényle, thiényle ou pyridyle, lesquels groupes peuvent être substitués sur leur cycle aromatique par 1, 2, 3 ou 4 substituants Y, où Y a la signification telle qu'indiquée ci-dessus, qui peuvent être identiques ou différents, ou R₇ représente un groupe alkyle en C_{1 à 8} ramifié ou linéaire, alcényle en C_{3 à 8}, cycloalkyle en C_{3 à 10,} bicycloalkyle en C_{5 à 10}, tricycloalkyle en C_{6 à 10} ou cycloalcényle en C_{5 à 8} ou R₇ représente un groupe naphtyle ou R₇ représente un groupe amino ou R₇ représente un groupe dialkylamino en C_{1 à 8}, un groupe monoalkylamino en C_{1 à 8} ou groupe hétérocyclique, monocyclique ou bicyclique saturé ou insaturé comportant 4 à 10 atomes de cycle, lequel groupe hétérocyclique contient 1 ou 2 atomes d'azote et lequel groupe hétérocyclique peut contenir un hétéroatome parmi le groupe (O, S) et lequel groupe hétérocyclique peut être substitué par un groupe alkyle en C_{1 à 3} ramifié ou linéaire, phényle, hydroxy ou trifluorométhyle ou un atome de fluor,
- R₈ représente un atome d'hydrogène ou un groupe méthyle,
- R₉ représente un atome d'hydrogène ou un groupe méthyle, éthyle, ou méthoxy,
et leurs tautomères, stéréoisomères et sels.

2. Composés selon la revendication 1 répondant à la formule générale (I) 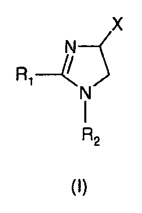 dans laquelle :
- R₁ et R₂ représentent indépendamment un groupe phényle, lequel groupe phényle peut être substitué par 1, 2 ou 3 substituants Y ayant la signification telle que donnée dans la revendication 1, ou R₁ et/ou R₂ représente un groupe naphtyle, thiényle ou pyridyle,
- X représente l'un des sous-groupes (i) ou (ii), 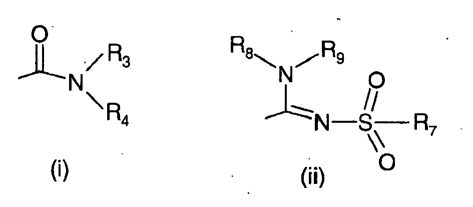
dans lesquels :
- R₃ représente un atome d'hydrogène,
- R₄ représente un groupe alkyle en C_{1 à 8} ramifié ou linéaire, alcoxy en C_{1 à 8} ramifié ou linéaire ou cycloalkyle en C_{3 à 8}, lesquels groupes peuvent être substitués par un groupe hydroxy, 1 à 3 groupes méthyle, un groupe éthyle, ou 1 à 3 atomes de fluor, ou R₄ représente un groupe phénoxy, pyridyle, thiényle ou R₄ représente un groupe NR₅R₆ où R₅ et R₆ - conjointement avec l'atome d'azote auquel ils sont attachés - forment un groupe hétérocyclique, monocyclique ou bicyclique saturé ou insaturé comportant 4 à 10 atomes de cycle, lequel groupe hétérocyclique contient un ou deux hétéroatomes parmi le groupe (O, N, S) ou
R₃ et R₄ - conjointement avec l'atome d'azote auquel ils sont attachés - forment un groupe hétérocyclique, monocyclique ou bicyclique saturé ou insaturé comportant 4 à 10 atomes de cycle, lequel groupe hétérocyclique contient un ou deux hétéroatomes parmi le groupe (O, N, S) et lequel groupe hétérocyclique peut être substitué par un groupe méthyle, hydroxy ou trifluorométhyle ou un atome de fluor,
R₇ représente un groupe phényle, lequel groupe phényle peut être substitué sur son cycle aromatique par 1, 2, 3 ou 4 substituants Y, où Y a la signification telle qu'indiquée ci-dessus, qui peuvent être identiques ou différents, ou R₇ représente un groupe alkyle en C_{1 à 8} ramifié ou linéaire, cycloalkyle en C_{3 à 10} ou bicycloalkyle en C_{5 à 10}, ou R₇ représente un groupe naphtyle ou R₇ représente un groupe amino ou R₇ représente un groupe dialkylamino en C_{1 à 8}, un groupe monoalkylamino en C_{1 à 8} ou un groupe hétérocyclique, monocyclique ou bicyclique saturé ou insaturé comportant 4 à 10 atomes de cycle, lequel groupe hétérocyclique contient 1 ou 2 atomes d'azote et lequel groupe hétérocyclique peut contenir un hétéroatome parmi le groupe (O, S) et lequel groupe hétérocyclique peut être substitué par un groupe alkyle en C_{1 à 3} ramifié ou linéaire, ou hydroxy,
- R₈ représente un atome d'hydrogène,
- R₉ représente un atome d'hydrogène
et leurs tautomères, stéréoisomères et sels.

3. Composé selon la revendication 1 qui est :
le 1-(4-chlorophényl)-2-(2,4-dichlorophényl)-N-(exo-2-bicyclo[2.2.1]heptyl)-4,5-dihydro-1H-imidazole-4-carboxamide (diastéréomère A)
le 1-(4-chlorophényl)-2-(2,4-dichlorophényl)-N-(exo-2-bicyclo[2.2.1]heptyl)-4,5-dihydro-1H-imidazole-4-carboxamide (diastéréomère B)
le 1-(4-chlorophényl)-2-(2,4-dichlorophényl)-N-(pipéridin-1-yl)-4,5-dihydro-1H-imidazole-4-carboxamide
le 1-(4-chlorophényl)-2-(2,4-dichlorophényl)-N-cyclohexyl-4,5-dihydro-1H-imidazole-4-carboxamide
la 1-(4-chlorophényl)-2-(2,4-dichlorophényl)-N-[(4-chlorophényl)sulfonyl]-4,5-dihydro-1 H-imidazole-4-carboxamidine
la 1-(4-chlorophényl)-2-(2,4-dichlorophényl)-N-[(4-fluorophényl)-sulfonyl]-4,5-dihydro-1 H-imidazole-4-carboxamidine
la 2-(4-chlorophényl)-N-(diméthylaminosulfonyl)-1-phényl-4,5-dihydro-1H-imidazole-4-carboxamidine
la 1-(4-chlorophényl)-2-(2,4-dichlorophényl)-N-(diméthylaminosulfonyl)-4,5-dihydro-1H-imidazole-4-carboxamidine

4. Compositions pharmaceutiques comprenant, en plus d'un véhicule pharmaceutiquement acceptable et/ou au moins une substance auxiliaire pharmaceutiquement acceptable, une quantité pharmacologiquement active d'au moins un composé selon l'une quelconque des revendications 1 à 3, ou un sel de celui-ci, en tant qu'ingrédient actif.

5. Composé selon l'une quelconque des revendications 1 à 3, ou un sel de celui-ci, pour une utilisation en médecine.

6. Utilisation d'un composé selon l'une quelconque des revendications 1 à 3, pour la préparation d'une composition pharmaceutique destinée au traitement de la psychose, de l'anxiété, de la dépression, de déficits de l'attention, de troubles de la mémoire, de troubles cognitifs, de troubles de l'appétit, de l'obésité, en particulier de l'obésité juvénile et de l'obésité induite par médicament, de la dépendance, de troubles du contrôle des impulsions, de l'appétence, de la dépendance aux médicaments et de troubles neurologiques tels que des troubles neurodégénératifs, la démence, la dystonie, la spasticité musculaire, le tremblement, l'épilepsie, la sclérose en plaques, une blessure cérébrale traumatique, un accident cérébro-vasculaire, la maladie de Parkinson, la maladie d'Alzheimer, l'épilepsie, la maladie de Huntington, le syndrome de Tourette, l'ischémie cérébrale, l'apoplexie cérébrale, un traumatisme craniocérébral, un accident cérébro-vasculaire, une blessure de la moelle épinière, des troubles neuro-inflammatoires, la sclérose en plaques, une encéphalite virale, des troubles apparentés à une démyélinisation, de même que destinée au traitement de troubles de la douleur, comprenant des troubles de la douleur neuropathique, et d'autres maladies impliquant une neurotransmission de cannabinoïde, comprenant le traitement du choc septique, du glaucome, d'un cancer, du diabète, des vomissements, des nausées, de l'asthme, de maladies respiratoires, de troubles gastro-intestinaux, d'ulcères gastriques, de diarrhées, de troubles cardio-vasculaires, de l'athérosclérose, de la cirrhose du foie et des troubles sexuels.

7. Utilisation d'un composé répondant à la formule (I) : 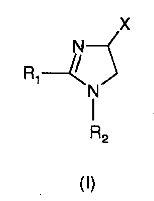 dans laquelle :
- R₁ et R₂ ont les significations telles que données dans la revendication 1, mais peuvent également représenter indépendamment un groupe méthylsulfonyle,
- X représente le sous-groupe (i), 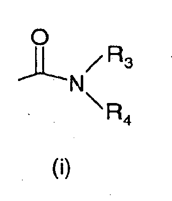
dans lequel :
- R₃ et R₄ ont les significations telles que données dans la revendication 1, mais dans lequel R₄ peut également représenter un groupe phényle, facultativement substitué par 1 à 3 substituants Y, où Y a la signification telle que donnée dans la revendication 1, pour la préparation d'une composition pharmaceutique destinée au traitement de la psychose, de l'anxiété, de la dépression, de déficits de l'attention, de troubles de la mémoire, de troubles cognitifs, de troubles de l'appétit, de l'obésité, en particulier de l'obésité juvénile et de l'obésité induite par médicament, de la dépendance, de troubles du contrôle des impulsions, de l'appétence, de la dépendance aux médicaments et de troubles neurologiques tels que des troubles neurodégénératifs, la démence, la dystonie, la spasticité musculaire, le tremblement, l'épilepsie, la sclérose en plaques, une blessure cérébrale traumatique, un accident cérébro-vasculaire, la maladie de Parkinson, la maladie d'Alzheimer, l'épilepsie, la maladie de Huntington, le syndrome de Tourette, l'ischémie cérébrale, l'apoplexie cérébrale, un traumatisme craniocérébral, un accident cérébro-vasculaire, une blessure de la moelle épinière, des troubles neuro-inflammatoires, la sclérose en plaques, une encéphalite virale, des troubles apparentés à une démyélinisation, de même que destinée au traitement de troubles de la douleur, comprenant des troubles de la douleur neuropathique, et d'autres maladies impliquant une neurotransmission de cannabinoïde, comprenant le traitement du choc septique, du glaucome, d'un cancer, du diabète, des vomissements, des nausées, de l'asthme, de maladies respiratoires, de troubles gastro-intestinaux, d'ulcères gastriques, de diarrhées, de troubles cardio-vasculaires, de l'athérosclérose, de la cirrhose du foie et des troubles sexuels.

8. Utilisation selon la revendication 6, **caractérisée en ce que** lesdits troubles sont des troubles de l'alimentation, en particulier l'obésité, l'obésité juvénile et l'obésité induite par médicament.

9. Utilisation d'un composé selon l'une quelconque des revendications 1 à 3, pour la préparation d'une composition pharmaceutique destinée au traitement de troubles de l'alimentation, en particulier l'obésité, l'obésité juvénile et l'obésité induite par médicament, **caractérisée en ce que** ladite composition pharmaceutique contient également au moins un inhibiteur de lipase.

10. Utilisation selon la revendication 9, **caractérisée en ce que** ledit inhibiteur de lipase est l'orlistat ou la lipstatine.
